# EUROPEAN PATENT APPLICATION

(11) **EP 4 316 508 A1**
(43) Date of publication of application: **07.02.2024**
(21) Application number: 22775775.4
(22) Date of filing: 24.03.2022
(51) Int. Cl.: A61K 38/54, A61K 9/14, A61K 9/19, A61K 31/198, A61K 47/26, A61K 47/36, A61K 49/00, A61P 27/04, A61P 31/04, C07C 315/06, C07C 317/48

(54) **DRIED PRENCSO COMPOSITION**

(30) Priority: 25.03.2021 JP 2021051876
(71) Applicant: House Foods Group Inc., Higashiosaka-shi, Osaka 577-8520 (JP)
(72) Inventor: UEYAMA Masae, Higashiosaka-shi, Osaka 577-8520 (JP); AOYAGI Morihiro, Higashiosaka-shi, Osaka 577-8520 (JP)
(74) Representative: Kordel, Mattias
(86) International application number: PCT/JP2022/013961
(87) International publication number: WO 2022/202994

(57) **Abstract**

The present specification discloses a dry PRENCSO composition improved in storage stability, and a lachrymatory factor generating kit comprising the composition. The dry PRENCSO composition according to the present disclosure comprises PRENCSO and saccharide. The present disclosure also relates to a method for producing a dry PRENCSO composition, the method comprising drying a solution containing PRENCSO and saccharide. The present disclosure also relates to a lachrymatory factor generating kit comprising the dry PRENCSO composition, and a dry enzyme composition comprising alliinase and a lachrymatory factor synthase (LFS) and being dried. The present disclosure also relates to a method for generating a lachrymatory factor by contacting water with the dry PRENCSO composition and the dry enzyme composition.

## Description

### Technical Field

The present invention relates to a dry PRENCSO composition.

The present invention also relates to a lachrymatory factor generating kit.

The present invention also relates to a method for generating a lachrymatory factor.

### Background Art

Thiopropanal S-oxide, a lachrymatory factor (LF) of onion, is generated by action of an enzyme alliinase on a precursor substance, PRENCSO (S-1-propenyl-cysteine sulfoxide), to generate sulfenic acid (E-1-propensulfenic acid), and isomerization of the sulfenic acid by a lachrymatory factor synthase (LFS).

The lachrymatory factor is known to be industrially useful compounds. For example, the lachrymatory factor is also known to be capable of being utilized as a lachrymal secretion test reagent and for a lachrymal secretion test method, in other words, a dry eye test (Patent Literature 1).

The lachrymatory factor is also known to be capable of being utilized in antimicrobial agents (Patent Literature 2).

The lachrymatory factor has low stability and are easily decomposed, and thus, when a lachrymatory factor is to be industrially utilized, the lachrymatory factor is preferably generated on the site where the lachrymatory factor is utilized.

Patent Literature 3 describes a lachrymatory factor generating kit. The lachrymatory factor generating kit described in Patent Literature 3 comprises a dry enzyme mixture, which is produced by allowing water-absorbing paper to absorb a solution containing alliinase and LFS and drying the resultant, and PRENCSO. Patent Literature 3 describes that PRENCSO is comprised in the kit as an acidic solution so as to be stably stored, and that a lachrymatory factor is generated by dropping the acidic solution of PRENCSO to the dry enzyme mixture, as specific embodiments.

### Citation List

### Patent Literature

Patent Literature 1: WO2005/067907
Patent Literature 2: JP2007-267639A
Patent Literature 3: JP2008-285476A

### Summary of Invention

### Technical Problem

In the method described in Patent Literature 3, which involves generating a lachrymatory factor by application of the PRENCSO acidic solution to the dry enzyme mixture, it is required to measure a determined amount of the PRENCSO acidic solution and then to apply this acidic solution to the dry enzyme mixture.

The present inventors have then studied about the utilization of PRENCSO as a dry composition, and thus have found the new problem that the dry composition of PRENCSO has a low storage stability.

The present invention is to solve the problem of non-sufficient storage stability of a dry PRENCSO composition. Additionally, an object of the present invention is to provide a lachrymatory factor generating kit comprising a dry PRENCSO composition with improved storage stability.

### Solution to Problem

The present inventors have found that a dry PRENCSO composition including PRENCSO and saccharide has better storage stability compared to a dry PRENCSO composition comprising no saccharide. The present specification discloses the following specific embodiments as solutions to solve the above problems.
[1] A dry PRENCSO composition comprising PRENCSO and saccharide, and being dried.
[2] The dry PRENCSO composition according to [1], comprising 5 parts by mass or more of saccharide relative to 1 part by mass of PRENCSO.
[3] The dry PRENCSO composition according to [1] or [2], further comprising a porous carrier carrying PRENCSO and saccharide.
[4] A method for producing a dry PRENCSO composition, the method comprising drying a solution containing PRENCSO and saccharide.
[5] The method according to [4], wherein the solution containing PRENCSO and saccharide comprises 5 parts by mass or more of saccharide relative to 1 part by mass of PRENCSO.
[6] The method according to [4] or [5], comprising allowing a porous carrier to absorb the solution containing PRENCSO and saccharide, and drying the resultant.
[7] A lachrymatory factor generating kit comprising:
   (1) the dry PRENCSO composition according to any of [1] to [3]; and
   (2) a dry enzyme composition comprising alliinase and a lachrymatory factor synthase (LFS), and being dried.
[8] The lachrymatory factor generating kit according to [7], wherein (1) and (2) are integrated.
[9] A method for generating a lachrymatory factor,
   comprising contacting water with
   (1) the dry PRENCSO composition according to any of [1] to [3], and
   (2) a dry enzyme composition comprising alliinase and a lachrymatory factor synthase (LFS), and being dried.
[10] The method according to [9], wherein (1) and (2) are integrated.

The present specification encompasses the disclosure of Japanese Patent Application No. 2021-051876 serving as the basis for the priority of the present application.

### Advantageous Effects of Invention

PRENCSO is in the form of the dry PRENCSO composition and thus is inhibited from being decomposed during storage.

A lachrymatory factor can be generated by contacting water with components of the lachrymatory factor generating kit.

### Brief Description of Drawings

[Figure 1] Figure 1 illustrates the measurement results of the amount of the remaining PRENCSO (%) in a PRENCSO pad comprising disaccharide as an additive, after storage at 40°C.
[Figure 2] Figure 2 illustrates the measurement results of the amount of the remaining PRENCSO (%) in a PRENCSO pad comprising saccharide alcohol as an additive, after storage at 40°C.
[Figure 3] Figure 3 illustrates the measurement results of the amount of the remaining PRENCSO (%) in a PRENCSO pad comprising monosaccharide as an additive, after storage at 40°C.
[Figure 4] Figure 4 illustrates the measurement results of the amount of the remaining PRENCSO (%) in a PRENCSO pad comprising polysaccharide as an additive, after storage at 40°C.
[Figure 5] Figure 5 is a plan view of a lachrymatory factor generating kit where a sheet-shaped dry PRENCSO composition and a sheet-shaped dry enzyme composition are stacked and integrated.
[Figure 6] Figure 6 is a cross-sectional view along with a line A-A in Figure 5, of the lachrymatory factor generating kit illustrated in Figure 5.
[Figure 7] Figure 7 is a plan view of a lachrymatory factor generating kit where a sheet-shaped dry PRENCSO composition and a sheet-shaped dry enzyme composition are adjacently located.
[Figure 8] Figure 8 is a cross-sectional view along with a line B-B in Figure 7, of the lachrymatory factor generating kit illustrated in Figure 7.
[Figure 9] Figure 9 is a plan view of a lachrymatory factor generating kit comprising one carrier obtained by integrating a porous carrier carrying PRENCSO and saccharide and a porous carrier carrying alliinase and LFS, and comprising a dry PRENCSO composition in which PRENCSO and saccharide are carried on one portion of the one carrier, and a dry enzyme composition in which alliinase and LFS are carried on another portion of the one carrier.
[Figure 10] Figure 10 is a cross-sectional view along with a line C-C in Figure 9, of the lachrymatory factor generating kit illustrated in Figure 9.

### Description of Embodiments

### <Dry PRENCSO composition and production method thereof>

One or more embodiments of the invention disclosed herein relate to a dry PRENCSO composition comprising PRENCSO and saccharide, and being dried. The dry PRENCSO composition according to the present disclosure, which comprises saccharide, is suitable for long-term storage because decomposition of PRENCSO during storage is suppressed, compared to a dry PRENCSO composition, which does not comprise saccharide.

Herein, the "dry" and "dried" are each not limited to absolute dry where no water is contained in a dry product at all, and encompass a state where about 0% by mass to 20% by mass of water is contained. In other words, preferably 20% by mass or less, more preferably 10% by mass or less, more preferably 3% by mass or less, more preferably 2% by mass or less of water may be contained relative to the total amount of a dry composition. The water content in each of the dry PRENCSO composition and a dry enzyme composition described below can be measured by absolutely drying such compositions and determining the proportion of the mass decreased by absolute drying, relative to the total mass of such compositions before absolute drying.

PRENCSO (S-1-propenyl-cysteine sulfoxide) may be artificially synthesized, or may be extracted from a natural product. PRENCSO purified by an ion-exchange resin or reversed-phase chromatography is preferably used.

The type of saccharide is not particularly limited, and, for example, one or more selected from monosaccharide, disaccharide, polysaccharide and sugar alcohol may be used. Each of monosaccharide, disaccharide, polysaccharide and sugar alcohol encompasses an ester thereof with fatty acid.

Examples of monosaccharide may include one or more selected from glucose and fructose. Examples of disaccharide may include one or more selected from trehalose, sucrose, sucrose ester, maltose and lactose. Examples of polysaccharide may include dextrin. Examples of sugar alcohol may include one or more selected from mannitol, isomaltulose (trade name Palatinose (registered trademark)) and a reduced product of isomaltulose.

The compounding ratio between PRENCSO and saccharide in the dry PRENCSO composition according to the present disclosure is not particularly limited, and preferably 5 parts by mass or more, more preferably 10 parts by mass or more, more preferably 50 parts by mass or more, more preferably 100 parts by mass or more of saccharide is comprised relative to 1 part by mass of PRENCSO. The upper limit of the compounding ratio is not particularly limited, and typically, preferably 1000 parts by mass or less, more preferably 500 parts by mass or less, more preferably 200 parts by mass or less of saccharide is comprised relative to 1 part by mass of PRENCSO. When multiple types of saccharide are compounded, the amount of saccharide in terms of the compounding ratio refers to the total amount of multiple types of saccharide.

The dry PRENCSO composition according to the present disclosure preferably comprises a porous carrier carrying PRENCSO and saccharide. The porous carrier is preferably a porous carrier being water absorptive, more preferably a porous carrier containing a hydrophilic polymer such as cellulose that is water absorptive, particularly preferably water absorptive paper. The shape of the porous carrier is not particularly limited, and is preferably a sheet shape. The dry PRENCSO composition, which comprises PRENCSO and saccharide, and the porous carrier carrying them, can be obtained by allowing the porous carrier to absorb a solution containing PRENCSO and saccharide, and drying the resultant.

The dry PRENCSO composition according to the present disclosure may also be in the form of powder or granule. The powder or granule comprising PRENCSO and saccharide may further comprise an adjuvant regularly used for formulation, such as an excipient, a binder, and a disintegrant. Examples of the excipient may include starch, milk sugar (lactose), white sugar (sucrose), methylcellulose, carboxymethylcellulose, sodium alginate, calcium hydrogen phosphate, synthetic aluminum silicate, microcrystalline cellulose, polyvinylpyrrolidone (PVP), and hydroxypropyl starch (HPS). Examples of the binder may include an aqueous solution or a water/ethanol solution of starch, microcrystalline cellulose, carboxymethylcellulose sodium, polyvinylpyrrolidone (PVP), powdered gum arabic, gelatin, glucose, white sugar, or the like. Examples of the disintegrant may include starch, carboxymethylcellulose, carboxymethylcellulose calcium, microcrystalline cellulose, hydroxypropyl starch, and calcium phosphate.

The dry PRENCSO composition according to the present disclosure preferably further comprises an acid such as hydrochloric acid. PRENCSO is stabilized in an acidic aqueous solution, as described in Patent Literature 3.

The method for producing the dry PRENCSO composition according to the present disclosure is preferably a method comprising drying a solution containing PRENCSO and saccharide. The solution containing PRENCSO and saccharide is preferably an aqueous solution, more preferably an acidic aqueous solution further containing an acid such as hydrochloric acid, particularly preferably an acidic aqueous solution having a pH of 3.5 or less. The compounding ratio between PRENCSO and saccharide in the solution may be the same as the compounding ratio in the dry PRENCSO composition. The solution may, if necessary, further comprise other component such as an adjuvant comprised in an objective composition.

A particularly preferable embodiment of the method for producing the dry PRENCSO composition according to the present disclosure comprises allowing the porous carrier to absorb the solution containing PRENCSO and saccharide, and drying the resultant. According to this embodiment, the dry PRENCSO composition, which comprises PRENCSO and saccharide, and the porous carrier carrying them, can be produced.

The drying method is not particularly limited, and lyophilization or hot-air drying may be exemplified.

The lyophilization is typically a method involving preliminarily freezing a sample at - 80°C to -20°C, and then drying it under reduced pressure. The drying temperature may be any appropriate temperature. Examples of the freezing include a method involving immersion in liquid nitrogen, a method involving loading into a freezer at -4°C to -80°C, and a method involving loading, together with dry ice, into an alcohol freezing bath, but not limited thereto. The drying temperature may be higher than the freezing temperature, and is preferably less than 80°C, more preferably less than 65°C, most preferably in the range of 20°C to 50°C.

The hot-air drying may be performed, for example, under a temperature condition of 40°C to 80°C, preferably about 60°C.

### <Method and kit for generating lachrymatory factor>

Other one or more embodiments of the invention disclosed herein relate to a method for generating a lachrymatory factor, comprising
contacting water with
(1) the dry PRENCSO composition according to the present disclosure, and
(2) a dry enzyme composition comprising alliinase and a lachrymatory factor synthase (LFS), and being dried.

Still other one or more embodiments of the invention disclosed herein relate to a lachrymatory factor generating kit comprising the dry PRENCSO composition of (1) above and the dry enzyme composition of (2) above.

In the method for generating a lachrymatory factor according to the present disclosure, by a simple operation in which the dry PRENCSO composition of (1) above and the dry enzyme composition of (2) above are allowed to co-exist and are contacted with water, enzyme alliinase acts on PRENCSO to generate sulfenic acid, and the sulfenic acid is further isomerized by a lachrymatory factor synthase (LFS) to generate a lachrymatory factor (LF) (thio propanal S-oxide). The method for generating a lachrymatory factor according to the present disclosure does not require a critical control of the amount of water to be contacted, and is thus easily carried out at the site where a lachrymatory factor is utilized. The method for generating a lachrymatory factor according to the present disclosure can be carried out with the lachrymatory factor generating kit according to the present disclosure.

Characteristics of the dry PRENCSO composition of (1) above are as described above.

Preferable embodiments of the dry enzyme composition of (2) above are described below.

As alliinase, alliinase derived from Alliaceae plants, particularly preferably alliinase derived from garlic, may be used. Alliinase may be purified alliinase or roughly purified alliinase. Roughly purified alliinase is extracted from, for example, garlic by a simple method. Examples of a simple method for preparing roughly purified alliinase include a "method involving adding an acid to an extracted solution of hydrated and pulverized garlic, and subjecting the resultant to isoelectric point precipitation of alliinase, followed by recovering and re-dissolving a precipitated product". The source for the extraction is not limited to garlic.

The dry enzyme composition may further comprise a protecting agent of alliinase. Examples of the protecting agent of alliinase include saccharide, particularly suitably include disaccharide. Examples of the disaccharide may include one or more disaccharides selected from sucrose, trehalose, maltose, lactose, or cellobiose. As the protecting agent of alliinase, a salt may further be comprised in addition to saccharide. Examples of the salt include any salt, for example, KCl, MgCl₂, and NaCl, and a monovalent metal salt is suitable and specifically NaCl is suitable. Further, the protecting agent of alliinase may also include pyridoxal phosphate. In other words, the protecting agent of alliinase preferably (i) comprises saccharide, or (ii) comprises saccharide, and a salt and/or pyridoxal phosphate.

As LFS may be LFS derived from plants belonging to the Alliaceae family (preferably onion), produced by a genetic recombination method, and a variant enzyme thereof, for example, LFS described in Domestic Re-publication of PCT International Publication WO02/020808, and Domestic Re-publication of PCT International Publication WO03/074707. As LFS, it is particularly preferable to use LFS prepared by a genetic recombination method described in Domestic Re-publication of PCT International Publication WO02/020808 (WO02/020808). LFS isolated from Alliaceae plants, in particular, an onion may also be used.

LFS may be purified LFS or roughly purified LFS. Roughly purified LFS may be, for example, LFS extracted from onion by a simple method. Examples of a simple method for preparing roughly purified LFS include a "method involving treating a hydrated and pulverized extracted solution of onion with an anion-exchange resin, and then performing adsorption and elution". The source of the extraction is not limited to an onion.

The compounding ratio between alliinase and LFS in the dry enzyme composition is not particularly limited, and, for example, alliinase may be comprised at 0.02 Units to 200 Units, more preferably 0.5 Units to 50 Units per microgram of LFS. LFS herein may be rLFS used in Examples, and has a specific activity of 6.0 × 10⁶ area/µg.

The dry enzyme composition preferably comprises a porous carrier carrying alliinase and LFS. The porous carrier is preferably a porous carrier being water absorptive, more preferably a porous carrier containing a hydrophilic polymer such as cellulose and being water absorptive, particularly preferably a water absorptive paper. The shape of the porous carrier is not particularly limited, and is preferably a sheet shape. The dry enzyme composition, which comprises alliinase and LFS, and the porous carrier carrying them, can be obtained by allowing the porous carrier to absorb a solution containing alliinase and LFS, and drying the resultant.

The dry enzyme composition may be in the form of powder or granule. The powder or granule comprising alliinase and LFS may further comprise an adjuvant regularly used for formulation, such as an excipient, a binder, and a disintegrant. Specific examples of the adjuvant are as described with respect to the dry PRENCSO composition.

The method for producing the dry enzyme composition preferably comprises drying a solution containing alliinase and LFS. The solution containing alliinase and LFS is preferably an aqueous solution, more preferably an aqueous solution further containing the protecting agent of alliinase. The compounding ratio between alliinase and LFS in the solution may be the same as the compounding ratio in the dry PRENCSO composition. The solution may, if necessary, further contain other component such as an adjuvant comprised in an objective composition.

A particularly preferable embodiment of the method for producing the dry enzyme composition comprises allowing the porous carrier to absorb the solution containing alliinase and LFS, and drying the resultant. According to this embodiment, the dry enzyme composition, which comprises alliinase and LFS, and the porous carrier carrying them, can be produced.

The drying method is not particularly limited, and lyophilization or hot-air drying may be exemplified. Preferable embodiments of lyophilization and hot-air drying are as described with respect to the dry PRENCSO composition.

The dry PRENCSO composition and the dry enzyme composition in the method for generating a lachrymatory factor and the lachrymatory factor generating kit according to the present disclosure are preferably integrated. With the dry PRENCSO composition and the dry enzyme composition being integrated in advance, a lachrymatory factor can be generated on the site where the lachrymatory factor is utilized, only by contacting the compositions with water, without any need for mixing both compositions. The lachrymatory factor generating kit according to the present disclosure, comprising the dry PRENCSO composition and the dry enzyme composition and in which the dry PRENCSO composition and the dry enzyme composition are integrated, can be referred to as a "lachrymatory factor generating tool" or "lachrymatory factor generating composition".

In a more preferable embodiment of an aspect where the dry PRENCSO composition and the dry enzyme composition are integrated, a dry PRENCSO composition further comprising a porous carrier carrying PRENCSO and saccharide, and a dry enzyme composition further comprising a porous carrier carrying alliinase and LFS are integrated so that water is permeable. In a further preferable embodiment thereof, the dry PRENCSO composition and the dry enzyme composition are integrated by being bound with an adhesive mean such as an adhesive or a water-permeable double-sided tape and thus integrated. It is particularly preferable in these embodiments that both the porous carrier carrying PRENCSO and saccharide and the porous carrier carrying alliinase and LFS have a sheet shape and the dry PRENCSO composition and the dry enzyme composition be stacked to be integrated.

In another aspect of the kit and the method according to the present disclosure, a dry PRENCSO composition further comprising a porous carrier carrying PRENCSO and saccharide and a dry enzyme composition further containing a porous carrier carrying alliinase and LFS are adjacently disposed. The kit according to this aspect can generate a lachrymatory factor when water is diffused entirely upon water being added to at least one of the dry PRENCSO composition and the dry enzyme composition.

In still another aspect of the kit and the method according to the present disclosure, in which a dry PRENCSO composition further comprising a porous carrier carrying PRENCSO and saccharide and a dry enzyme composition further comprising a porous carrier carrying alliinase and LFS, the porous carrier carrying PRENCSO and saccharide and the porous carrier carrying alliinase and LFS are integrated in one carrier, and PRENCSO and saccharide are carried on one portion of the one carrier and alliinase and LFS are carried on another portion of the one carrier. In the kit according to this aspect, one portion of the one carrier, on which PRENCSO and saccharide are carried, corresponds to the dry PRENCSO composition, and another portion of the one carrier, on which alliinase and LFS are carried, corresponds to the dry enzyme composition. The kit according to this aspect can generate a lachrymatory factor when water is diffused entirely upon water being added to the one carrier.

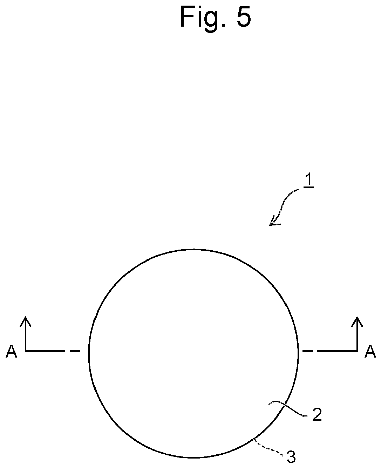

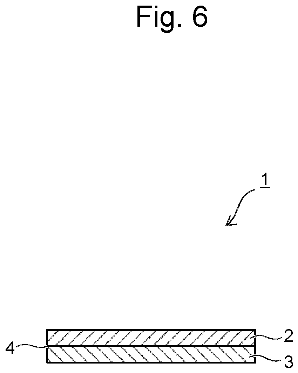

Figure 5 and Figure 6 illustrate one example of a lachrymatory factor generating kit (lachrymatory factor generating tool), in which a dry PRENCSO composition comprising PRENCSO and saccharide and a porous carrier carrying them and a dry enzyme composition containing alliinase and LFS and a porous carrier carrying them are stacked to be integrated. In a lachrymatory factor generating kit 1 in this example, a sheet-shaped dry PRENCSO composition 2 and a sheet-shaped dry enzyme composition 3 are stacked to be integrated. In the lachrymatory factor generating kit 1 in this example, the dry PRENCSO composition 2 and the dry enzyme composition 3 are preferably stacked so that water is permeable in a boundary section 4 between these compositions. Although not illustrated, an adhesive mean such as an adhesive or a water-permeable double-sided tape may be interposed between the dry PRENCSO composition 2 and the dry enzyme composition 3 in the boundary section 4.

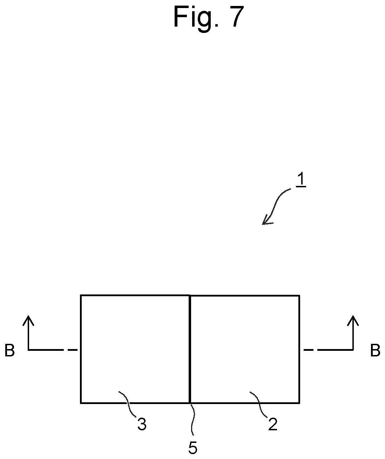

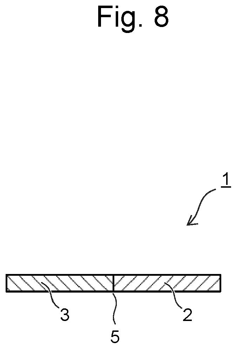

Figure 7 and Figure 8 illustrate one example of a lachrymatory factor generating kit, in which a dry PRENCSO composition containing PRENCSO and saccharide and a porous carrier carrying them and a dry enzyme composition containing alliinase and LFS and a porous carrier carrying them are adj acently located. In a lachrymatory factor generating kit 1 in this example, a sheet-shaped dry PRENCSO composition 2 and a sheet-shaped dry enzyme composition 3 are adjacently located on one surface. In the lachrymatory factor generating kit 1 in this example, water is preferably permeable in a boundary section 5 between the dry PRENCSO composition 2 and the dry enzyme composition 3. The dry PRENCSO composition 2 and the dry enzyme composition 3 may or may not be bound to each other in the boundary section 5.

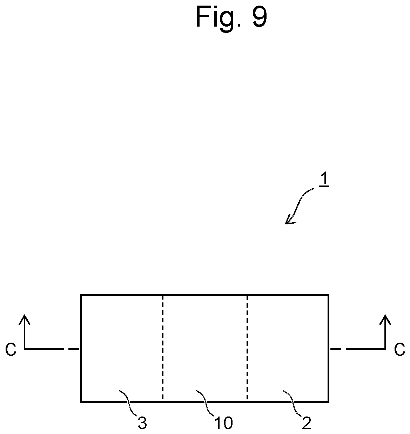

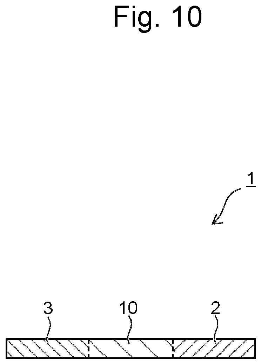

Figure 9 and Figure 10 illustrate one example of a lachrymatory factor generating kit comprising one carrier, in which a porous carrier carrying PRENCSO and saccharide and a porous carrier carrying alliinase and LFS are integrated, and comprising the dry PRENCSO composition carrying PRENCSO and saccharide on one portion of the one carrier, and the dry enzyme composition carrying alliinase and LFS on another portion of the one carrier. The lachrymatory factor generating kit 1 in this example comprises the dry PRENCSO composition 2 corresponding to portion where PRENCSO and saccharide are carried on one portion of a sheet-shaped carrier 10, and the dry enzyme composition 3 where alliinase and LFS are carried on another portion of the sheet-shaped carrier 10.

In a case where the dry PRENCSO composition and the dry enzyme composition are in the form of granule or powder, one example of "being integrated" also includes cases where the dry PRENCSO composition and the dry enzyme composition are mixed.

The water to be contacted with the dry PRENCSO composition and the dry enzyme composition, for generation of a lachrymatory factor, in the method for generating a lachrymatory factor and the lachrymatory factor generating kit according to the present disclosure may be pure water or an aqueous solution containing other component in the water. Examples of such other component include a surfactant. In a case where an aqueous solution of a surfactant is contacted with the dry PRENCSO composition and the dry enzyme composition, a lachrymatory factor tends to be more easily generated compared to the case of using pure water. The surfactant is preferably a non-ionic surfactant or a zwitterionic surfactant, particularly preferably a non-ionic surfactant. Examples of the non-ionic surfactant may include polysorbate 20, polysorbate 60, polysorbate 65, and polysorbate 80. The surfactant is preferably added to the water so as to have a final concentration of 0.005% by mass to 1% by mass. The lachrymatory factor generating kit according to the present disclosure may comprise an aqueous solution containing the surfactant, as a further constituent component.

The subject for the method for generating a lachrymatory factor and the lachrymatory factor generating kit according to the present disclosure is not limited to humans. Specifically, the method may also be applied to mammals such as a monkey, a dog, a cat, a horse, a cow, a sheep, a rabbit, and a mouse.

The method for exposing a LF (lachrymatory factor) to the eye is not particularly limited, and is most efficiently to place the lachrymatory factor generating kit in a lachrymatory cup and to fill the cup with LF, and then the one eye of the subject is closely attached so that the eye is covered with an opening. In this regard, LF having volatility has only to be applied to the eye, and thus tears are also induced by setting the lachrymatory factor generating kit under the eye. The lachrymatory factor generating kit may be set under the eye by directly attaching the lachrymatory factor generating kit under the eye or by using a tool for installing the lachrymatory factor generating kit under the eye, such as goggles.

### Examples

### 1. Method for preparing PRENCSO pad

### 1.1. Materials

20 mg/ml PRENCSO solution
0.1 N hydrochloric acid
Various saccharides (disaccharide, sugar alcohol, monosaccharide, polysaccharide)
Thin circular paper filter (ϕ8 mm, thickness 0.7 mm)
-80°C Freezer
Lyophilizer

### 1.2. Method

(1) A PRENCSO solution was prepared by diluting a 20 mg/ml PRENCSO solution and each additive with ultrapure water so that the final concentration of PRENCSO was 1 mg/ml and the final concentration of each additive was as shown in the following Tables.

In the case of adding hydrochloric acid, a PRENCSO solution was prepared by dilution with a mixed solution, in that ultrapure water: 0.1 N hydrochloric acid (15:1, v/v), instead of ultrapure water.

**[Table 1]**

| Disaccharide | Final concentration |
|---|---|
| D-(+)-Trehalose dihydrate | 100 mg/ml |
| D-(+)-Trehalose dihydrate/Hydrochloric acid | 100 mg/ml |
| Sucrose | 100 mg/ml |
| Sucrose/Hydrochloric acid | 100 mg/ml |
| Sucrose + | 5 mg/ml |
| Sucrose ester | 5 µg/ml |
| Sucrose + | 5 mg/ml |
| Sucrose ester/Hydrochloric acid | 5 µg/ml |
| Maltose | 100 mg/ml |
| Maltose/Hydrochloric acid | 100 mg/ml |
| Lactose | 100 mg/ml |
| Lactose/Hydrochloric acid | 100 mg/ml |

**[Table2]**

| Sugar alcohol | Final concentration |
|---|---|
| Reduced palatinose | 100 mg/ml |
| Reduced palatinose/Hydrochloric acid | 100 mg/ml |
| D-(-)-Mannitol | 100 mg/ml |

**[Table3]**

| Monosaccharide | Final concentration |
|---|---|
| Glucose | 100 mg/ml |
| Glucose/Hydrochloric acid | 100 mg/ml |
| Fructose | 100 mg/ml |
| Fructose/Hydrochloric acid | 100 mg/ml |

**[Table4]**

| Polysaccharide | Final concentration |
|---|---|
| Tapioca starch dextrin | 100 mg/ml |
| Tapioca starch dextrin/Hydrochloric acid | 100 mg/ml |
| Pinedex #6 | 100 mg/ml |
| Pinedex #6/Hydrochloric acid | 100 mg/ml |

(2) PRENCSO control/hydrochloric acid was prepared by diluting a 20 mg/ml PRENCSO solution with a mixed solution, in that ultrapure water: 0.1 N hydrochloric acid (15:1, v/v), so that the final concentration of PRENCSO was 1 mg/ml.

(3) Each PRENCSO solution was applied by 30 µl to a circular paper filter, and frozen at -80°C.

(4) After freezing, a PRENCSO pad was obtained by drying with a lyophilizer for 14 hours or more.

### 2. PRENCSO pad storage test

### 2.1. Materials

PRENCSO pad
1.5-ml Tube
Aluminum pouch
Thin circular paper filter (ϕ8 mm, thickness 0.7 mm)

### 2.2. Apparatuses

Constant-temperature bath (ADVANTEC PE-315T)
Trace centrifuge
0.2-µm Filter
1-ml Syringe
Waters Alliance HPLC, or Thermo Ultimate 3000 HPLC

### 2.3. High-performance liquid chromatography (HPLC) analysis conditions

Column ODS (Senshu Scientific Co., Ltd.)
Detection 230 nm
Oven temperature 35°C
Amount of injection 20 µl
Flow rate 0.6 ml/min
Mobile phase (MeOH: TFA water (pH 3.3) = 30%:70%, isocratic) or (MeOH: TFA water (pH 3.3) = 3%:97%, isocratic)
Sample cooler 5°C

### 2.4. Method

(1) The PRENCSO pad produced was placed in a 1.5-ml tube, and tightly sealed, and furthermore placed in an aluminum pouch and tightly sealed.
(2) The resultant was left in a constant-temperature bath at 40°C.
(3) The amount of PRENCSO in the PRENCSO pad stored in the constant-temperature bath at 40°C for arbitrary period was determined. To the PRENCSO pad after storage for a predetermined number of days, 580 µl of water was added, and the resultant was tightly stoppered and mixed by a vortex mixer for 30 seconds.
(4) A control (= an amount of PRENCSO of 100%, a number of storage days of 0 day) was obtained by placing a circular paper filter in a 1.5-ml tube, allowing 30 µl of a 1 mg/ml PRENCSO (+ additive) solution, which has frozen and thawed, to permeate, followed by adding 550 µl of water and tightly stoppered, and then mixing the resultant by a vortex mixer for 30 seconds.
(5) Centrifuged at 4°C for 3 minutes.
(6) 200 to 300 µl of the supernatant was filtered, and transferred to a HPLC vial.
(7) The peak area of PRENCSO at a detection wavelength of 230 nm and a retention time of about 6.4 minutes was determined with HPLC.

The proportion of the peak area of PRENCSO in a sample from the PRENCSO pad after storage for the predetermined period, relative to the peak area of control PRENCSO in each condition, was defined as the amount of the remaining PRENCSO (%).

### 3. Results

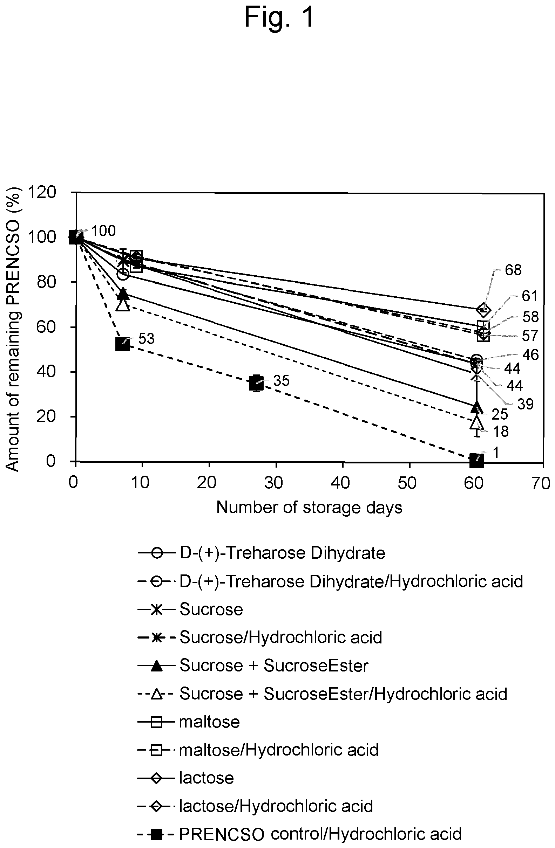

Figure 1 illustrates the measurement results of the amount of the remaining PRENCSO (%) in a PRENCSO pad comprising disaccharide as an additive, after storage at 40°C.

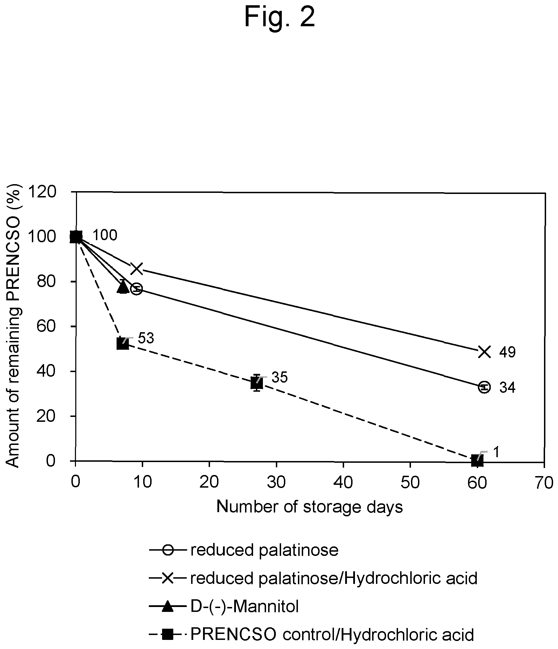

Figure 2 illustrates the measurement results of the amount of the remaining PRENCSO (%) in a PRENCSO pad comprising sugar alcohol as an additive, after storage at 40°C.

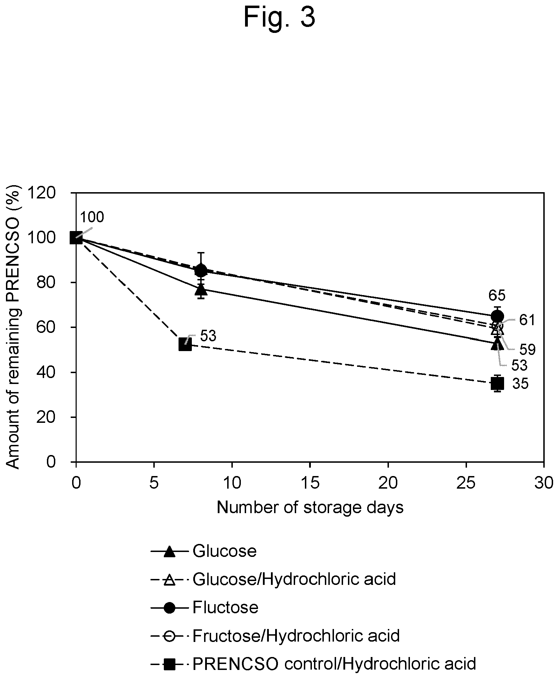

Figure 3 illustrates the measurement results of the amount of the remaining PRENCSO (%) in a PRENCSO pad comprising monosaccharide as an additive, after storage at 40°C.

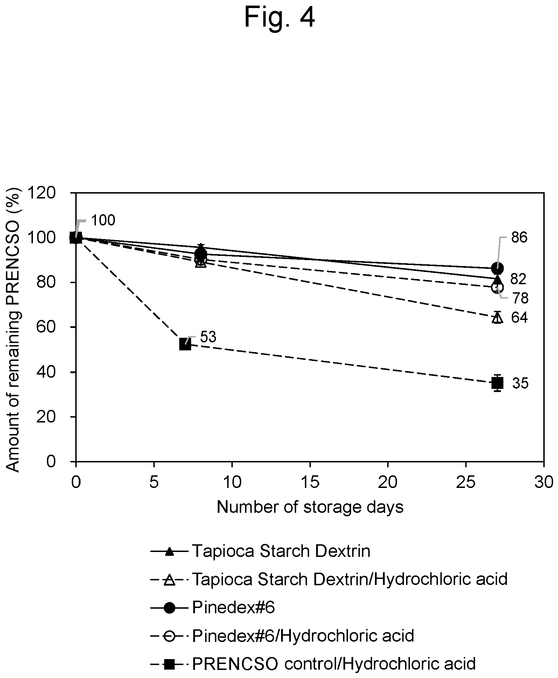

Figure 4 illustrates the measurement results of the amount of the remaining PRENCSO (%) in a PRENCSO pad comprising polysaccharide as an additive, after storage at 40°C.

### 4. Method for preparing enzyme pad

### (1) Preparation of LFS

A lachrymatory factor synthase (LFS) was produced by a genetic recombination method, according to the method described in WO02/020808.

The outline was as follows. A plasmid (pGEX-4T-3-E2-3-1) expressing LFS protein was introduced to E.coli, by a competent method, in order to obtain a transformant. The transformant obtained was subjected to shaking culture at 37°C in an LB medium containing 100 µg/ml of ampicillin. When isopropyl-β-thiogalactopyranoside (IPTG) was added for the induced production to the medium, a fusion protein of glutathione S transferase (GST) and E2-3-1 (hereinafter, referred to as "GST-E2-3") was accumulated in the body of the bacteria.

The transformant was cultured as described above and the body of the bacteria was collected by centrifugation, and then ultrasonically crushed. The supernatant recovered by centrifugation was allowed to flow in a glutathione sepharose 4 Fast Flow column (manufactured by Amersham Pharmacia Biotech, Inc.), and a GST fusion protein was adsorbed to the column. After the column was washed, the fusion protein was eluted with an elution buffer containing reduced glutathione, and a purified E2-3 fusion protein sample was obtained.

The fusion protein sample was allowed to flow in a HiTrap Desalting column (manufactured by Amersham Pharmacia Biotech, Inc.), to remove the reduced glutathione, and adsorbed to the glutathione sepharose 4 Fast Flow column again. After the column was washed, the column was filled with a buffer containing thrombin, and subjected to protease treatment at room temperature for 2 hours, to cleave a GST tag from the fusion protein. Recombinant E2-3 from which the GST tag was removed was eluted from the column, and then Benzamidine Sepharose 6 was added to the eluate and mixed, and centrifuged to remove thrombin in the eluate, and thus a recombinant E2-3 sample was obtained (which is referred to as rLFS).

### (2) Preparation of alliinase

### (2-1) Pulverization/acid precipitation of garlic

First, a pot of mixer was placed in a refrigerator to cool. A rotor was installed in a low-temperature centrifuge, and cooled at a temperature set to 4°C. An equivalent of a buffer A was added to a garlic piece (100 g), and the piece was pulverized by the mixer. A duplicate gauze on an opening of the pot placed on ice was fastened with an elastic band, and the pulverized product was allowed to flow onto the gauze to filter. After a certain amount of the filtrate was obtained, the pulverized product on the gauze was enclosed with the gauze, and squeezed. The filtrate obtained was centrifuged at 4°C and at 12000 rpm for 10 minutes, and a centrifuge supernatant was recovered. While the centrifuge supernatant recovered was stirred with being placed on ice, acetic acid was added with the pH being monitored, and a pH of 4.0 is achieved. After a pH of 4.0 was achieved, the resultant was left for 5 minutes, as it was. A sample with a precipitate emerged was centrifuged at 4°C and at 12000 rpm for 10 minutes, and a pellet centrifuged was recovered (with the buffer A).

The pellet recovered was adjusted to be at a volume of 50 ml to 100 ml (buffer A), and was left at 5°C for 30 minutes, as it was. The sample was centrifuged at 4°C and at 12000 rpm for 10 minutes. The centrifuge supernatant recovered was prepared with an aqueous 1 N sodium hydroxide solution so as to have a pH of 6.5. This is referred to as a rough extracted solution of alliinase.

### (2-2) Treatment with hydroxyapatite column

The rough extracted solution of alliinase (centrifuge supernatant) was applied to a hydroxyapatite column equilibrated with the buffer A. The solution was adsorbed in the form of a yellow band, to the hydroxyapatite column. The hydroxyapatite column to which such a sample was applied was washed with 300 ml of the buffer A. 300 ml of a buffer C was allowed to flow in the washed hydroxyapatite column for elution. The eluate was fractionated by 10 ml with a fraction collector, and a fraction in which a yellow eluate was fractionated was collected.

### (2-3) Purification of alliinase with ConA column

A 20 mM calcium chloride/magnesium chloride solution in an amount of 1/20 of the amount of the fraction collected (20 ml) was added to increase the concentrations of calcium and magnesium ions in a sample solution. Re-generation was made thereafter, and the resultant was applied to a ConA column equilibrated with a starting buffer (a recommended buffer described in the manual of ConA Sepharose 4B). The ConA column after application of the sample was washed with 50 ml of the starting buffer. 50 ml of a ConA elution buffer (a recommended buffer described in the manual of ConA Sepharose 4B) was allowed to flow in the washed ConA column for elution. The eluate was fractionated by 2 ml with a fraction collector, and a fraction in which a yellow eluate was fractionated was collected.

### (2-4) Method for concentrating alliinase purified

10 ml of the yellow eluate obtained by ConA column purification (alliinase solution) was introduced in CENTRIPLUS CONCENTRATORS (up to 15 ml, No. 4421) (manufactured by Merck Millipore). CENTRIPLUS CONCENTRATORS was installed in a centrifuge cooled to 4°C, and centrifuged at 3000 rpm for 30 minutes, the content was confirmed once, and then centrifuged once again at 3000 rpm for 30 minutes. The activity of the alliinase solution with concentration completed was measured according to the following method, and confirmed to have achieved the required concentration.

The buffer A (pH 7.0) (50 mM buffer A for alliinase purification) was prepared as follows. A 50 mM dipotassium hydrogen phosphate solution (5.22 g dissolved in 600 ml) and a 50 mM potassium dihydrogen phosphate solution (3.4 g dissolved in 500 ml) were prepared. Both were mixed with the pH being monitored, and the pH was adjusted to 7.0. To 9 volumes of the resulted buffer, 1 volume of glycerol was added and mixed well. To 1 L of the resulted glycerol-containing buffer, 5.3 mg of pyridoxal phosphate was added and mixed. The resulted buffer was labelled, and stored in a low-temperature experiment depot (10°C).

The buffer C (pH 7.0) (500 mM buffer C for alliinase purification) was prepared as follows. A 500 mM dipotassium hydrogen phosphate solution (43.6 g dissolved in 500 ml) and a 500 mM potassium dihydrogen phosphate solution (34.0 g dissolved in 500 ml) were prepared. Both were mixed with the pH being monitored, and the pH was adjusted to 7.0. To 9 volumes of the resulted buffer, 1 volume of glycerol was added and mixed well. To 1 L of the resulted glycerol-containing buffer, 5.3 mg of pyridoxal phosphate was added and mixed. The resulted buffer was labelled, and stored in a low-temperature experiment depot (10°C).

### (2-5) Method for measuring activity of alliinase

The activity of alliinase was calculated by a method for measuring the amount of pyruvic acid, which is produced in decomposition of PRENCSO by alliinase.

A 100 mM phosphate buffer (pH 6.5) was used to prepare each of a 0.16 mg/ml pyridoxal phosphate solution, a 3.8 mg/ml NADH solution, a 6.0 µg/ml lactase dehydrogenase (LDH) solution, and 20 mg/ml PRENCSO solution. Using the above pyridoxal phosphate solution, an alliinase solution was diluted 100-fold to prepare a sample. 100 µl of a 0.16 mg/ml pyridoxal phosphate solution, 100 µl of a 3.8 mg/ml NADH solution, 100 µl of a 6.0 µg/ml LDH solution, and 100 µl of a 20 mg/ml PRENCSO solution were added to 2500 µl of 100 mM phosphate buffer (pH 6.5), and these were mixed. To this mixed solution, 100 µl of an alliinase solution, as a specimen, was added to initiate the enzyme reaction, and the change in absorbance of the reaction solution over time was measured for 3 minutes immediately after the start of the reaction. The decreasing rate in absorbance was calculated from the obtained result of the change in absorbance to confirm the decreasing rate in absorbance falling within the range of 0.02 to 0.19. If the decreasing rate in absorbance did not fall within this range, re-measurement was performed with the rate of dilution of the alliinase solution being changed. The alliinase activity (Unit) was calculated from the obtained decreasing rate in absorbance, namely, the amount of change in absorbance per minute.

### (3) Preparation of enzyme pad

To 120 µl of a 0.02 mg/ml rLFS solution, 120 µl of a 50 U/ml alliinase solution (10% trehalose, 1% NaCl, 25 µM pyridoxal phosphate) was added and mixed. This mixed solution was absorbed by 30 µl by a thin circular paper filter (ϕ8 mm, thickness 0.7 mm), and then preliminarily frozen at -80°C under atmospheric pressure. After freezing, lyophilization was performed at -10°C under reduced pressure to obtain an enzyme pad.

### 5. Method for preparing integrated lachrymatory factor generating kit

The PRENCSO pad and the enzyme pad each produced were bonded with a tape paste (DOTLINER from KOKUYO Co., Ltd.) to integrate, and thus the integrated lachrymatory factor generating kit was obtained.

All publications, patents, and patent applications cited herein are herein incorporated by reference as they are.

## Claims

1. A dry PRENCSO composition comprising PRENCSO and saccharide, and being dried.

2. The dry PRENCSO composition according to claim 1, comprising 5 parts by mass or more of saccharide relative to 1 part by mass of PRENCSO.

3. The dry PRENCSO composition according to claim 1 or 2, further comprising a porous carrier carrying PRENCSO and saccharide.

4. A method for producing a dry PRENCSO composition, the method comprising drying a solution containing PRENCSO and saccharide.

5. The method according to claim 4, wherein the solution containing PRENCSO and saccharide comprises 5 parts by mass or more of saccharide relative to 1 part by mass of PRENCSO.

6. The method according to claim 4 or 5, comprising allowing a porous carrier to absorb the solution containing PRENCSO and saccharide, and drying the resultant.

7. A lachrymatory factor generating kit comprising:
(1) the dry PRENCSO composition according to any one of claims 1 to 3; and
(2) a dry enzyme composition comprising alliinase and a lachrymatory factor synthase (LFS), and being dried.

8. The lachrymatory factor generating kit according to claim 7, wherein (1) and (2) are integrated.

9. A method for generating a lachrymatory factor, comprising contacting water with
(1) the dry PRENCSO composition according to any one of claims 1 to 3, and
(2) a dry enzyme composition comprising alliinase and a lachrymatory factor synthase (LFS), and being dried.

10. The method according to claim 9, wherein (1) and (2) are integrated.
